(19) **Europäisches Patentamt / European Patent Office / Office européen des brevets**

(11) **EP 4 759 359 A1**

(12) **EUROPÄISCHE PATENTANMELDUNG**

(43) Veröffentlichungstag:
**17.06.2026  Patentblatt 2026/25**

(21) Anmeldenummer: **24218686.4**

(22) Anmeldetag: **10.12.2024**

(51) Internationale Patentklassifikation (IPC):
***A61N 7/00*** (2006.01)

(52) Gemeinsame Patentklassifikation (CPC):
**A61N 7/00;** A61N 2007/0034; A61N 2007/0073

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Wellcomet GmbH**
**76646 Bruchsal (DE)**

(72) Erfinder: **KRUGLIKOV, Ilja**
**76351 Linkenheim-Hochstetten (DE)**

(74) Vertreter: **LBP Lemcke, Brommer & Partner**
**Patentanwälte mbB**
**Siegfried-Kühn-Straße 4**
**76135 Karlsruhe (DE)**

(54) **SYSTEM ZUR ERZEUGUNG VON ULTRASCHALLWELLEN**

(57)  Vorgeschlagen wird ein System (1) zur Erzeugung von Ultraschallwellen, insbesondere zur kosmetischen oder therapeutischen Behandlung, aufweisend:
einen elektrisch angesteuerten Ultraschallkopf (5), der dazu ausgebildet ist, Ultraschallwellen $U(f_i)$ mit mehreren unterschiedlichen Frequenzen $f_i$ zu erzeugen, wobei die genannten unterschiedlichen Frequenzen $f_i$ eine Gruppe von Frequenzen bilden,
einen Signalgenerator (4), der dazu ausgebildet ist, Signale $S(f_i)$ mit den genannten Frequenzen $f_i$ zu erzeugen und zur Umwandlung in Ultraschallwellen $U(f_i)$ an dem Ultraschallkopf (5) bereitzustellen,
und
eine Steuereinheit (3), die dazu ausgebildet ist, permanent oder zumindest während vorgegebener Zeitspannen zwischen den Frequenzen $f_i$ umzuschalten.
Das System (1) zeichnet sich dadurch aus, dass die Steuereinheit (3) dazu ausgebildet ist, nach Maßgabe einer Zufallsfunktion (6) zwischen allen oder zumindest einigen der genannten Frequenzen $f_i$ zufällig umzuschalten, wobei die einigen der genannten Frequenzen $f_i$ eine Untergruppe der genannten Gruppe bilden.

Fig. 1

EP 4 759 359 A1

**Beschreibung**

Die Erfindung betrifft ein System zur Erzeugung von Ultraschallwellen nach dem Oberbegriff des Anspruchs 1.

Aus der EP 3 031 439 B1 ist ein gattungsgemäßes System bekannt, bei dem Ultraschallwellen zur Behandlung biologischen Gewebes eingesetzt werden. Das System zeichnet sich durch die Fähigkeit aus, zwischen mindestens zwei vorgegebenen Frequenzen schnell umzuschalten, wobei die höhere Frequenz für eine längere Zeitspanne angewendet wird als die niedrigere. Dies reduziert unerwünschte Effekte wie Periostenentzündungen oder Schmerzen. Die verwendeten Frequenzen, die typischerweise in einem Bereich von 0,7 MHz bis 20 MHz liegen, ermöglichen eine flexible Anpassung für tiefe oder oberflächliche Gewebebehandlungen.

Besonders hervorzuheben ist beim Gegenstand der EP 3 031 439 B1 die Frequenzwahl in nicht ganzzahligen Verhältnissen, die die Effizienz zwischen elektrischer Eingangs- und akustischer Ausgangsleistung optimiert. Das System nutzt dabei eine Steuerung zur individuellen Anpassung der Behandlungsparameter und kann eine Kombination mit anderen Therapien umfassen. Dies führt zu einer verbesserten Mikromassagewirkung, die für verschiedene Anwendungen bei entzündlichen und hyperproliferativen Hauterkrankungen sowie für Anti-Aging Behandlungen vorteilhaft ist.

Für eine genauere Beschreibung der mit Ultraschall bei der Behandlung von Gewebe erreichbaren vorteilhaften Wirkungen darf auf die Offenbarung in der o.g. Druckschrift verwiesen werden, die durch Bezugnahme mit in die vorliegende Anmeldung aufgenommen wird. Wie die weiteren Untersuchungen und Veröffentlichungen der Anmelderin gezeigt haben, wird die Hauptwirkung dieser Wellen durch eine Modulation des Signalproteins Caveolin-1 realisiert, welches als Target für Entzündliche und hyperproliferative Hauterkrankungen sowie für Anti-Aging Korrekturen dient.

Obwohl sich dieser Ansatz in der Praxis bewährt hat, besteht Bedarf an einer Weiterentwicklung des vorbekannten Systems, die sich durch noch bessere Wirksamkeit auszeichnet und insbesondere Gewöhnungseffekten vorbeugen soll.

Diese Aufgabe wird erfindungsgemäß gelöst durch ein System mit den Merkmalen des Anspruchs 1. Vorteilhafte Weiterbildungen sind in den Unteransprüchen definiert.

Ein erfindungsgemäßes System zur Erzeugung von Ultraschallwellen, insbesondere zur kosmetischen oder therapeutischen Behandlung, weist auf: einen elektrisch angesteuerten Ultraschallkopf, der dazu ausgebildet ist, Ultraschallwellen mit mehreren unterschiedlichen Frequenzen $f_i$ zu erzeugen, wobei die genannten unterschiedlichen Frequenzen $f_i$ eine Gruppe von Frequenzen bilden; einen Signalgenerator, der dazu ausgebildet ist, Signale $S(f_i)$ mit den genannten Frequenzen $f_i$ zu erzeugen und zur Umwandlung in Ultraschallwellen $U(f_i)$ an dem Ultraschallkopf bereitzustellen; und eine Steuereinheit, die dazu ausgebildet ist, permanent oder zumindest während vorgegebener Zeitspannen zwischen den Frequenzen $f_i$ umzuschalten. Das erfindungsgemäße System ist dadurch gekennzeichnet, dass die Steuereinheit dazu ausgebildet ist, nach Maßgabe einer Zufallsfunktion zwischen allen oder zumindest einigen der genannten Frequenzen $f_i$ zufällig umzuschalten, wobei die einigen der genannten Frequenzen $f_i$ eine Untergruppe der genannten Gruppe bilden. Auf diese Weise ergibt sich eine zufällige oder stochastische Frequenzabfolge.

Die Zufallsfunktion kann in an sich bekannterweise mittels eines Random Number Generators (Zufallszahlengenerators) implementiert sein, was dem Fachmann an sich geläufig ist.

Hierdurch wird insbesondere erreicht, dass es aufgrund des zufälligen Auswählens zumindest einer Unteranzahl der möglichen Frequenzen $f_i$ nicht zu Gewöhnungseffekten kommt. Auf diese Weise kann mit einer gleichen Ultraschall-Leistung wie bei vorbekannten Geräten eine verbesserte Wirkung erzielt werden.

Folgende Weiterbildungen der Erfindung haben sich in vorklinischen Untersuchungen als besonders vorteilhaft erwiesen:
Bei einer Ausgestaltung des erfindungsgemäßen Systems ist vorgesehen, dass die genannte Gruppe von Frequenzen $f_i$ eine Anzahl von mindestens drei, vorzugsweise mindestens fünf, höchst vorzugsweise mindestens zehn Frequenzen $f_i$ umfasst.

Mit einer solchen Anzahl von Frequenzen ist nach Erkenntnissen der Anmelderin eine effiziente zufällige (stochastische) Frequenzabfolge realisierbar. Außerdem kann eine Auswahl der Frequenzen aus einem (relativ) breiten Frequenzspektrum eine stärkere Wirkung auf das genannte Protein Caveolin-1 entfalten, was sich als günstig erwiesen hat.

Bei einer anderen Ausgestaltung des erfindungsgemäßen Systems ist vorgesehen, dass die Frequenzen $f_i$ in einem Bereich zwischen 3 MHz und 19 MHz, vorzugsweise in einem Bereich zwischen 5 MHz und 15 MHz, liegen. Dabei können die Frequenzen möglichst gleichmäßig über den Bereich verteilt angeordnet sein.

Die Anmelderin hat erkannt, dass sich speziell bei den genannten Frequenzen bzw. in dem entsprechenden Frequenzbereich die bestmöglichen Ergebnisse bei der bevorzugten Verwendung der Erfindung zum Behandeln (speziell zum Verbessern des Hautbildes) von menschlicher Haut erzielen lassen. Dies schließt insbesondere die Behandlung von Narbengewebe und Wunden mit ein.

Bei einer weiteren Ausgestaltung des erfindungsgemäßen Systems ist vorgesehen, dass der Ultraschallkopf einen Ultraschallwandler (Transducer) aufweist, vorzugsweise einen sog. Dickenschwinger, der etwa bei einer mittleren Frequenz aus der genannten Gruppe, vorzugsweise bei einer Frequenz von etwa 10 MHz, seine Resonanzfrequenz oder Grundschwingungsfrequenz besitzt. Der Ultraschallwandler, vorzugsweise ein Piezoelement, dient dazu, ein elektronisches Anregungssignal in mechanische Ultraschallschwingungen bzw. -Wellen umzuwandeln, was dem Fachmann an sich wohlbekannt ist.

Bei noch einer weiteren Ausgestaltung des erfindungsgemäßen Systems ist vorgesehen, dass die Steuereinheit dazu ausgebildet ist, nach Maßgabe der Zufallsfunktion zwischen allen oder zumindest einigen der genannten Frequenzen $f_i$ mit Ausnahme der genannten Resonanzfrequenz zufällig umzuschalten und die genannte Resonanzfrequenz nach einem regelmäßigen Muster zu wiederholen, insbesondere bei jedem zweiten oder dritten Frequenzwechsel.

Auf diese Weise ist die Erfindung in der Lage, den Effekt der sog. stochastischen Resonanz auszunutzen, der besagt, dass ein Wirk-Signal effektiv verstärkt werden kann, wenn gleichzeitig zu dem Wirk-Signal ein Rausch-Signal (bei Frequenzen abseits des Wirk-Signals) vorhanden ist. Vorliegend wird das Signal bei der genannten mittleren Frequenz als das Wirk-Signal angesehen; die Signale bei den genannten anderen Frequenzen $f_i$ mit Ausnahme der genannten Resonanzfrequenz bilden eine Art Rauschen.

Bei noch einer anderen Ausgestaltung des erfindungsgemäßen Systems ist vorgesehen, dass die Steuereinheit dazu ausgebildet ist, nach jeweils einer fest vorgegebenen oder einer ebenfalls zufällig gewählten Umschaltzeit zwischen den Frequenzen $f_i$ umzuschalten. Die Umschaltzeit, insbesondere die zufällig gewählte Umschaltzeit, kann dabei zwischen 0,1 ms und 50 ms, vorzugsweise zwischen 0,1 ms und 10 ms, betragen.

Bei derart kurzen Umschaltzeiten, was einer ebenso langen Erzeugungsdauer der betreffenden Frequenzen entspricht, kann eine menschliche Gewebezellen nicht mehr sukzessive auf die durch die Ultraschallwellen hervorgerufenen Schwingungsphänomene reagieren. Die entsprechende Reaktionszeit der Zellen beträgt nach Erkenntnissen der Anmelderin etwa 100 ms bis 500 ms. Deshalb kommt es bei den genannten relativ kurzen Umschaltzeiten zu einer sog. "biologischen Interferenz", bei der die Zellen auf verschiedene Frequenzen gleichzeitig reagieren müssen und sich nicht auf die Ultraschalleinwirkung einer Frequenz einstellen können. Dies wird als besonders hilfreich für einen guten Behandlungserfolg angesehen. Nach Erkenntnissen der Anmelderin ist der genannte Effekt der "biologischen Interferenz" bei Umschaltzeiten kleiner 10 ms bzw. zwischen 0,1 ms und 10 ms besonders ausgeprägt. Eine schnelle Umschaltung der Frequenzen erlaubt eine quasi-simultane Anwendung von allen Frequenzen aus einem breiten Frequenzspektrum, was die Behandlungseffektivität erhöht und Gewöhnungseffekte bei einzelnen Frequenzen reduziert.

Bei wieder einer anderen Ausgestaltung des erfindungsgemäßen Systems ist vorgesehen, dass die Umschaltzeit mit der Frequenz korreliert ist, wobei höhere Frequenzen längere Umschaltzeiten aufweisen. Dies ist gleichbedeutend damit, dass höhere Frequenzen bzw. die entsprechenden Ultraschallwellen während einer längeren Zeitdauer erzeugt bzw. angelegt werden.

Die Anmelderin hat erkannt, dass sich auch eine solche Ausgestaltung günstig auf den erzielbaren Behandlungserfolg auswirken kann.

Bei einer bevorzugten Ausgestaltung des erfindungsgemäßen Systems ist die Steuereinheit dazu ausgebildet, bei einer der Frequenzen eine erste Erzeugungsdauer auszuwählen und bei den anderen Frequenzen eine von der ersten Erzeugungsdauer verschiedene, zweite Erzeugungsdauer auszuwählen. Dabei kann vorzugsweise die zweite Erzeugungsdauer kürzer sein als die erste Erzeugungsdauer. Weiterhin kann höchst vorzugsweise die eine der Frequenzen der oben eingeführten Resonanzfrequenz entsprechen. Außerdem beträgt insbesondere die erste Erzeugungsdauer 10 ms und die zweite Erzeugungsdauer zwischen 0,1 ms und 1,0 ms.

Die Erzeugungsdauer entspricht der o.g. Umschaltzeit: Wenn nach einer Zeit T von einer Frequenz auf die nächste Frequenz umgeschaltet wird, wird die eine Frequenz mit einer Erzeugungsdauer T erzeugt.

Auf diese Weise lassen sich die weiter oben angesprochenen Effekte der stochastischen Resonanz, der biologischen Interferenz nebst der effizienten Anregung von Harmonischen und Sub-Harmonischen in beliebiger Kombination und jeweiliger Ausprägung nutzen.

Des Weiteren hat es sich als vorteilhaft erwiesen, wenn in Weiterbildung des erfindungsgemäßen Systems die Steuereinheit dazu ausgebildet ist, zusammen mit den Frequenzen $f_i$ auch eine Intensität der Ultraschallwellen zu verändern, wobei vorzugsweise die Intensität aus einem Intervall gewählt ist, das von 0 bis 3 $W/cm^2$, vorzugsweise von 0,5 bis 1,5 $W/cm^2$, reicht.

Grundsätzlich lässt sich nach Erkenntnissen der Anmelderin ein guter Behandlungserfolg speziell dann erzielen, wenn die Intensität über das gesamte verwendete Frequenzspektrum möglichst gleichmäßig verteilt ist, wobei sich die genannten Werte als besonders vorteilhaft erwiesen haben.

Außerdem hat es sich als vorteilhaft erwiesen, wenn in Weiterbildung des erfindungsgemä-ßen Systems die Steuereinheit dazu ausgebildet ist, die Intensität zumindest für einige der Frequenzen nach Maßgabe einer Zufallsfunktion zu verändern, so dass sich eine zufällige Intensitätsabfolge ergibt. Auf diese Weise lassen sich insbesondere die vorteilhaften Eigenschaften der bereits mehrfach erwähnten stochastischen Resonanz im Rahmen der Erfindung nutzen.

Dieser Nutzung wird noch verstärkt, wenn bei einer anderen Weiterbildung des erfindungsgemäßen Systems die Steuereinheit dazu ausgebildet ist, bei einer der Frequenzen eine erste Intensität auszuwählen und bei den anderen Frequenzen eine von der ersten Intensität verschiedene, zweite Intensität auszuwählen.

Die Anmelderin hat auch herausgefunden, dass sich gute Behandlungsergebnisse erzielen lassen, wenn bei einer Weiterbildung des erfindungsgemäßen Systems die Steuereinheit dazu ausgebildet ist, alle Frequenzen mit im Wesentlichen gleicher Wahrscheinlichkeit oder Häufigkeit, p, auszuwählen, $p \propto f°$. Dies wird vorliegend auch als eine Art weißes Rauschen angesehen und gilt bei entsprechender statistischer Betrachtungsweise, also nach einer Vielzahl an Umschaltvorgängen, ggf. mit Ausnahme der genannten Resonanzfrequenz, die nach einem abweichenden Muster verwendet werden kann. Außerdem kann dieser Ansatz auch für die Ultraschallintensität und/oder für die Erzeugungs- bzw. Umschaltzeiten gelten: auch diese können aus einem jeweiligen Werteintervall zufällig und mit der genannten Häufigkeit durch die Steuereinheit ausgewählt werden.

Die Anmelderin hat weiterhin herausgefunden, dass sich bei einigen Indikationen gute Behandlungsergebnisse erzielen lassen, wenn bei einer Weiterbildung des erfindungsgemäßen Systems die Steuereinheit dazu ausgebildet ist, die Frequenzen mit einer Wahrscheinlich oder Häufigkeit, p, auszuwählen, die einer Gauß'schen Normalverteilung mit einem Maximum bei einer der Frequenzen entspricht. Auch dies gilt bei entsprechender statistischer Betrachtungsweise, also nach einer Vielzahl an Umschaltvorgängen, ggf. mit Ausnahme der genannten Resonanzfrequenz, die nach einem abweichenden Muster verwendet werden kann. Außerdem kann dieser Ansatz wiederum auch für die Ultraschallintensität und/oder für die Erzeugungs- bzw. Umschaltzeiten gelten: auch diese können aus einem jeweiligen Werteintervall zufällig und mit der genannten Häufigkeit durch die Steuereinheit ausgewählt werden.

Die Anmelderin hat schließlich auch herausgefunden, dass sich bei einigen Indikationen gute Behandlungsergebnisse erzielen lassen, wenn bei einer Weiterbildung des erfindungsgemä-ßen Systems die Steuereinheit dazu ausgebildet ist, die Frequenzen mit einer Wahrscheinlich oder Häufigkeit, p, auszuwählen, die einer von dem Frequenzwert abhängigen mathematischen Funktion entspricht, wobei vorzugweise gilt $p \propto 1/f$ oder $p \propto f^{-2}$ oder $p \propto f$ oder $p \propto f^2$. Dies entspricht einer Art von farbigem Rauschen. Auch dies gilt bei entsprechender statistischer Betrachtungsweise, also nach einer Vielzahl an Umschaltvorgängen, ggf. mit Ausnahme der genannten Resonanzfrequenz, die nach einem abweichenden Muster verwendet werden kann. Außerdem kann dieser Ansatz wiederum auch für die Ultraschallintensität und/oder für die Erzeugungs- bzw. Umschaltzeiten gelten: auch diese können aus einem jeweiligen Werteintervall zufällig und mit der genannten Häufigkeit durch die Steuereinheit ausgewählt werden.

Kombinationen der vorstehend beschriebenen Ansätze sind ebenfalls möglich: z.B. können die Frequenzen gemäß einer ersten Art von Rauschen zufällig ausgewählt werden, während die Intensitäten und/oder die Umschaltzeiten fest vorgegeben und/oder nach der ersten oder einer zweiten Art von Rauschen zufällig ausgewählt werden. Während einer Behandlung können die Einstellungen auch verändert werden, um Gewöhnungseffekte noch sicherer zu vermeiden.

Weitere Eigenschaften und Vorteile der Erfindung ergeben sich aus der folgenden Beschreibung konkreter Ausführungsbeispiele anhand der Zeichnung.

Figur 1 zeigt eine schematische Darstellung des erfindungsgemäßen Systems;

Figur 2 zeigt eine erste Ausgestaltung der stochastischen Frequenzauswahl;

Figur 3 zeigt eine zweite Ausgestaltung der stochastischen Frequenzauswahl;

Figur 4 zeigt eine dritte Ausgestaltung der stochastischen Frequenzauswahl;

Figur 5 zeigt eine vierte Ausgestaltung der stochastischen Frequenzauswahl;

Figur 6 zeigt eine erste Ausgestaltung der Auswahl von Intensität und/oder Umschaltzeit in Abhängigkeit von der Frequenz;

Figur 7 zeigt eine zweite Ausgestaltung der Auswahl von Intensität und/oder Umschaltzeit in Abhängigkeit von der Frequenz;

Figur 8 zeigt eine dritte Ausgestaltung der Auswahl von Intensität und/oder Umschaltzeit in Abhängigkeit von der Frequenz; und

Figur 9 zeigt eine vierte Ausgestaltung der Auswahl von Intensität und/oder Umschaltzeit in Abhängigkeit von der Frequenz.

In allen Figuren stehen gleiche Bezugszeichen für gleiche oder zumindest gleichwirkende Elemente.

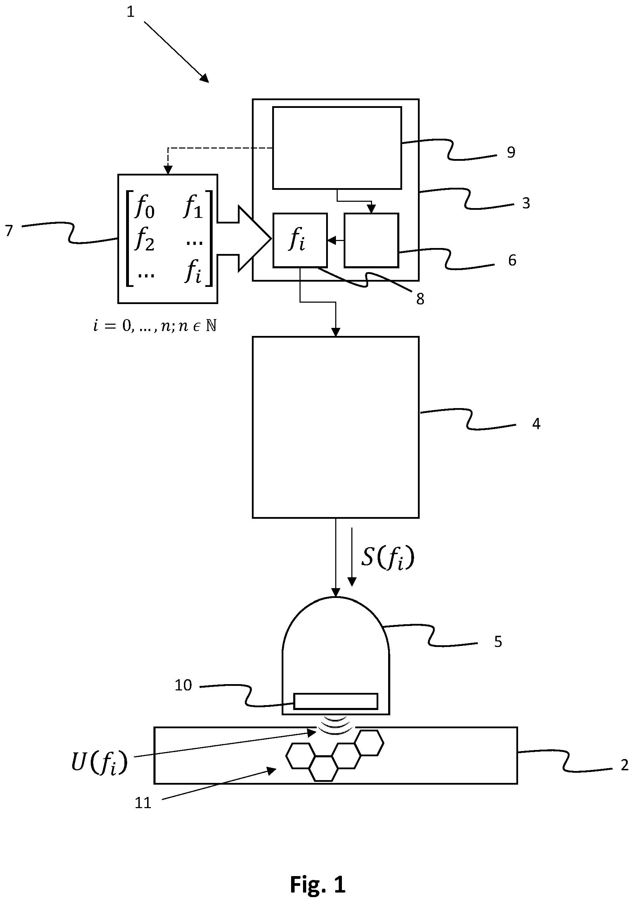

In der Figur 1 ist eine schematische Darstellung des erfindungsgemäßen Systems 1 gezeigt. Es dient zur Erzeugung von Ultraschallwellen, insbesondere zur kosmetischen oder therapeutischen Behandlung von Körpergewebe 2, und umfasst eine Steuereinheit 3, die mit einem Signalgenerator 4 wirkverbunden ist, der wiederum seinerseits mit einem elektrisch angesteuerten Ultraschallkopf 5 wirkverbunden ist. Mittels des Ultraschallkopfes 5 wird auf das Gewebe eingewirkt, wie schematisch dargestellt.

Der Ultraschallkopf 5 ist dazu ausgebildet, Ultraschallwellen $U(f_i)$ mit mehreren unterschiedlichen Frequenzen $f_i$ zu erzeugen, wobei die genannten unterschiedlichen Frequenzen $f_i$ ein Gruppen von Frequenzen bilden. Der Signalgenerator 4 ist dementsprechend dazu ausgebildet, Signale $S(f_i)$ mit den genannten Frequenzen $f_i$ zu erzeugen und zur Umwandlung in Ultraschall-

wellen $U(f_i)$ an dem Ultraschallkopf 5 bereitzustellen. Die Steuereinheit 3 ist dazu ausgebildet, permanent oder zumindest während vorgegebener Zeitspannen zwischen den Frequenzen $f_i$ umzuschalten. Dazu beinhaltet die Steuereinheit 3 eine Zufallsfunktion bzw. die (softwaretechnische) Implementierung 6 einer solchen, nach deren Maßgabe zwischen allen oder zumindest einigen der genannten Frequenzen $f_i$ zufällig, d.h. stochastisch umgeschaltet werden kann. Die möglichen Frequenzen $f_i$ sind vorgegeben, wie bei Bezugszeichen 7 schematisch dargestellt ist (mit $i = 0, ..., n$; $n \in \mathbb{N}$ ), und in einem Speichermittel 8 der Steuereinheit 3 abgelegt. Der Wert für n kann beispielsweise 10 betragen. Die Steuereinheit 3 weist außerdem noch eine Benutzerschnittstelle 9 auf, vorzugsweise nach Art eines GUI (Graphical User Interface), über die der Benutzer Einstellungen und Veränderungen an den Frequenzen $f_i$ vornehmen (gestrichelter Pfeil in Figur 1) oder und/oder die zufällige Frequenzausgabe veranlassen kann. Die zuvor erwähnten einigen der genannten Frequenzen $f_i$ bilden eine Untergruppe der genannten Gruppe.

Nach Maßgabe durch die Zufallsfunktion bzw. deren Implementierung 6 ergibt sich so im Betrieb des Systems 1 eine zufällige Frequenzabfolge, was eine entsprechend zufällige (stochastische) Beaufschlagung des Gewebes 2 mit Ultraschallwellen zur Folge hat.

Wie die Figur 1 noch zeigt, umfasst der Ultraschallkopf 5 zur Erzeugung der Ultraschallwellen $U(f_i)$ einen Ultraschallwandler 10 (sog. Transducer), der als Dickenschwinger ausgebildet sein kann. Der Ultraschallwandler 10 empfängt die Frequenzsignale $S(f_i)$ von dem Signalgenerator 4 und erzeugt daraus die Ultraschallwellen $U(f_i)$ mit der jeweiligen Frequenz, Intensität und Dauer, wobei diese Parameter von der Ansteuerung durch den Signalgenerator 4 abhängen und vorliegend (durch den Benutzer) einstellbar sind, insbesondere nach Maßgabe durch bzw. über die Steuereinheit 3.

Das Gewebe 2 umfasst Zellen 11, die erfindungsgemäß vorzugsweise derart mit (Ultraschall-) Schwingungen $U(f_i)$ beaufschlagt werden, dass an ihnen die bereits angesprochene biologische Interferenz auftritt, um die gewünschten Behandlungseffekte zu verstärken. Besonders bevorzugt ist eine zumindest teilweise stochastische Beaufschlagung mit Ultraschallwellen mit einer Frequenz zwischen 5 MHz und 15 MHz während einer Dauer von jeweils weniger als 10 ms (1 ms bis 10 ms) und einer zugehörigen Intensität von 1 W/cm² bis 1,5 W/cm².

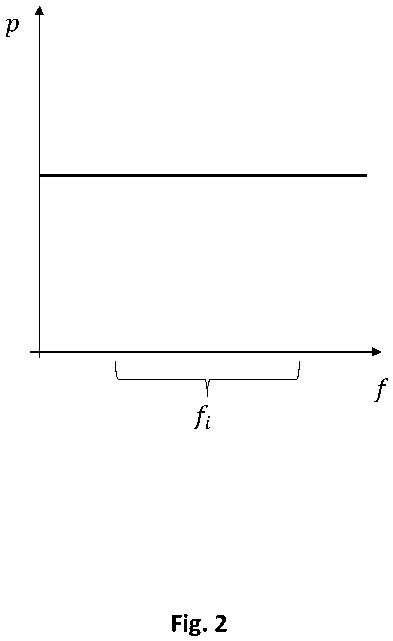

Die folgenden Figuren 2 bis 9 zeigen steuerungstechnische Möglichkeiten zur Realisierung der im Rahmen der Erfindung Verwendung findenden stochastischen Frequenzauswahl.

Die Graphen in den Figuren 2 bis 5 zeigen die (numerische) Häufigkeit p, mit der eine bestimmte Frequenz $f_i$ ausgewählt wird.

Figur 2 illustriert den bereits angesprochenen Fall, bei dem die Steuereinheit 3 (siehe Figur 1) dazu ausgebildet ist, die Frequenzen $f_i$ mit einer Wahrscheinlich oder Häufigkeit, p, auszuwählen, die analog zu einem weißen Rauschen ausgebildet ist: $p \propto f^0$. Dies entspricht einer horizontalen Linie im f-p-Diagramm. Das markierte Intervall $f_i$ bezeichnet den Bereich, aus dem die Frequenzen $f_i$ ausgewählt werden, z.B. zwischen 5 MHz und 15 MHz. Mit anderen Worten: alle Frequenzen $f_i$ werden statistisch gesehen mit gleicher Wahrscheinlichkeit bzw. Häufigkeit ausgewählt.

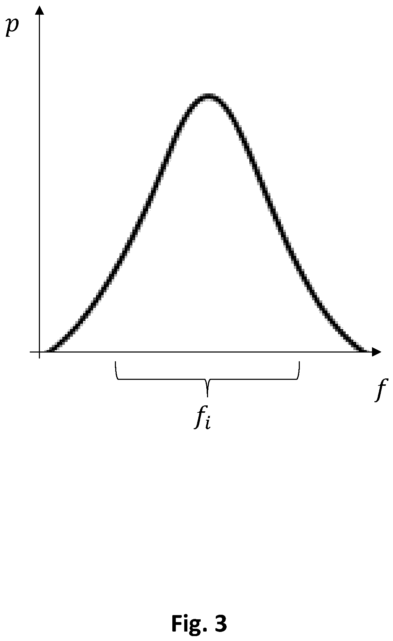

Figur 3 illustriert den bereits angesprochenen Fall, bei dem die Steuereinheit 3 (siehe Figur 1) dazu ausgebildet ist, die Frequenzen $f_i$ mit einer statistischen Wahrscheinlich oder Häufigkeit, p, auszuwählen, die einer Gauß'schen Normalverteilung mit einem Maximum bei einer der Frequenzen $f_i$ entspricht, z.B. der mittleren Frequenz aus dem markierten Intervall, insbesondere bei einer Frequenz von 10 MHz. Das markierte Intervall $f_i$ bezeichnet wiederum den Bereich, aus dem die Frequenzen $f_i$ ausgewählt werden, z.B. zwischen 5 MHz und 15 MHz. Mit anderen Worten: die Frequenzen $f_i$ werden statistisch gesehen mit einer Wahrscheinlichkeit bzw. Häufigkeit ausgewählt, die der gezeigten Gauß'schen Glockenkurve entspricht.

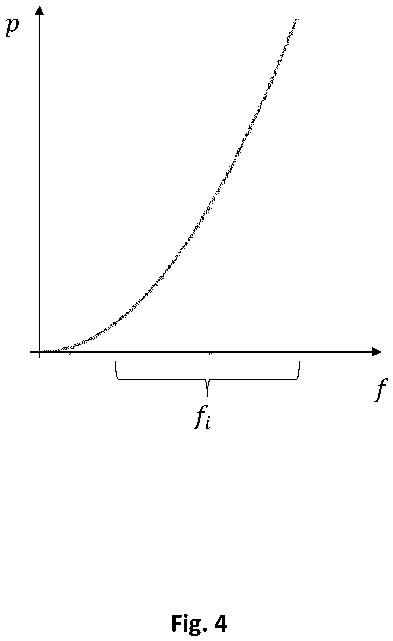

Figur 4 illustriert den bereits angesprochenen Fall, bei dem die Steuereinheit 3 (siehe Figur 1) dazu ausgebildet ist, die Frequenzen $f_i$ mit einer statistischen Wahrscheinlich oder Häufigkeit, p, auszuwählen, die einer von dem Frequenzwert abhängigen mathematischen Funktion entspricht, wobei gemäß Figur 4 gilt: $p \propto f^2$. Das markierte Intervall $f_i$ bezeichnet erneut den Bereich, aus dem die Frequenzen $f_i$ ausgewählt werden, z.B. zwischen 5 MHz und 15 MHz. Mit anderen Worten: die Frequenzen $f_i$ werden statistisch gesehen mit einer Wahrscheinlichkeit bzw. Häufigkeit ausgewählt, die gemäß der gezeigten Parabelkurve von der Frequenz abhängt.

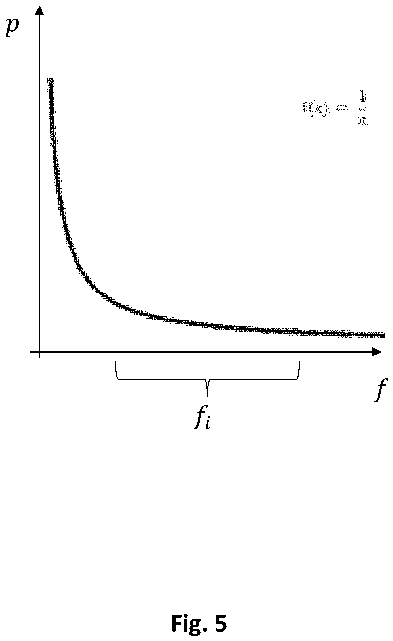

Figur 5 illustriert den bereits angesprochenen Fall, bei dem die Steuereinheit 3 (siehe Figur 1) dazu ausgebildet ist, die Frequenzen $f_i$ mit einer statistischen Wahrscheinlich oder Häufigkeit, p, auszuwählen, die einer von dem Frequenzwert abhängigen mathematischen Funktion entspricht, wobei gemäß Figur 4 gilt: $p \propto f^1$. Das markierte Intervall $f_i$ bezeichnet auch hier den Bereich, aus dem die Frequenzen $f_i$ ausgewählt werden, z.B. zwischen 5 MHz und 15 MHz. Mit anderen Worten: die Frequenzen $f_i$ werden statistisch gesehen mit einer Wahrscheinlichkeit bzw. Häufigkeit ausgewählt, die gemäß der gezeigten Kurve von der Frequenz abhängt. Auch eine Abhängigkeit $p \propto f^2$ ist möglich (nicht gezeigt).

Grundsätzlich können die Frequenzen aber auch derart ausgewählt werden, dass es keinen eindeutigen formalen Zusammenhang der genannten Art zwischen Häufigkeit/Wahrscheinlichkeit p auch Frequenz f gibt.

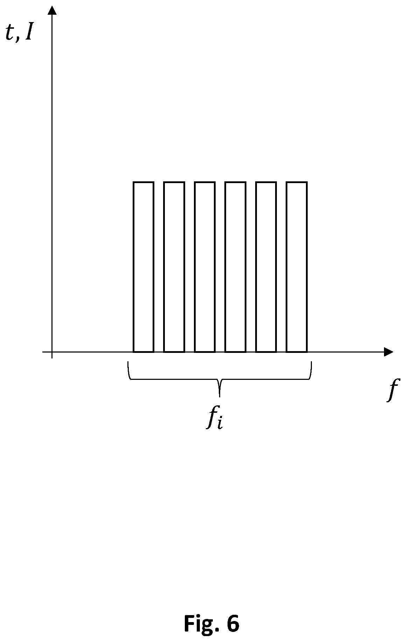

Die Graphen in den Figuren 6 bis 9 zeigen die Intensität I der Ultraschallwellen $U(f_i)$ und deren Erzeugungsdauer t in Abhängigkeit von der Frequenz f.

In der Figur 6 sind die Intensitätswerte bzw. Zeitdauern pro Frequenz als Balken gleicher Höhe und mit endlicher Breite dargestellt, obwohl in der Praxis bevorzugt jeweils genau ein exakter Frequenzwert ausgewählt wird. Das markierte Intervall $f_i$ bezeichnet auch hier den Bereich, aus dem die Frequenzen $f_i$ ausgewählt werden, z.B. zwischen 5 MHz und 15 MHz.

Die genannten Zeitdauern betragen bevorzugt weniger als 10 ms (z.B. 1 ms bis 10 ms), und die Intensität liegt bevorzugt zwischen 1 W/cm$^2$ und 1,5 W/cm$^2$.

Mit anderen Worten: Figur 6 zeigt (ohne Beschränkung) sechs distinkte Frequenzwerte bzw. entsprechend erzeugte Ultraschallwellen $U(f_i)$, die alle während einer festgelegten gleichen Zeitdauer und mit gleicher festgelegter Intensität erzeugt werden, sofern sie innerhalb der stochastischen Frequenzauswahl zufällig ausgewählt werden. Dies lässt sich im Rahmen der Erfindung beliebig mit den Häufigkeiten gemäß einer der Figuren 2 bis 5 kombinieren.

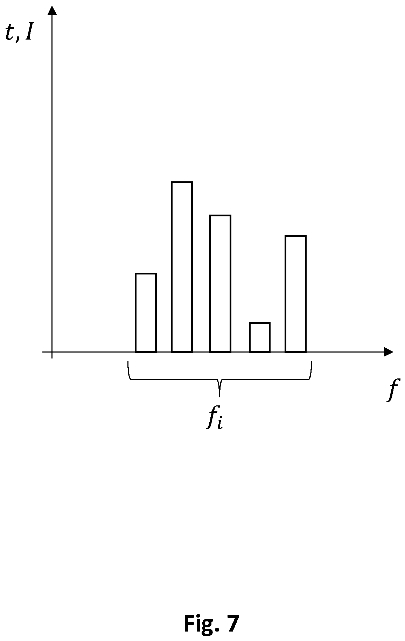

Die gezeigte Ausgestaltung kann natürlich auch auf nur einen der beiden Parameter Intensität und Zeitdauer beschränkt werden, während der jeweils andere Parameter konstant gehalten oder in anderer Weise modifiziert wird. Dies gilt in gleicher Weise für die Ausgestaltungen gemäß den Figuren 7ff. Die kombinierte zeichnerische Darstellung soll nur die Anzahl der erforderlichen Figuren reduzieren.

In der Figur 7 sind die Intensitätswerte bzw. Zeitdauern pro Frequenz als Balken unterschiedlicher Höhe und mit gleicher, endlicher Breite dargestellt, obwohl in der Praxis wiederum bevorzugt jeweils genau ein exakter Frequenzwert ausgewählt wird. Das markierte Intervall $f_i$ bezeichnet auch hier den Bereich, aus dem die Frequenzen $f_i$ ausgewählt werden, z.B. zwischen 5 MHz und 15 MHz. Die genannten Zeitdauern betragen bevorzugt weniger als 10 ms (z.B. 1 ms bis 10 ms), und die Intensität liegt bevorzugt zwischen 1 W/cm$^2$ und 1,5 W/cm$^2$.

Mit anderen Worten: Figur 7 zeigt (ohne Beschränkung) fünf distinkte Frequenzwerte bzw. entsprechend erzeugte Ultraschallwellen $U(f_i)$, die alle während einer unterschiedlichen Zeitdauer und/oder mit jeweils unterschiedlicher Intensität erzeugt werden. Die Auswahl von Intensität und/oder Zeitdauer kann zufällig erfolgen, bevorzugt nach Maßgabe durch die Zufallsfunktion 6 gemäß Figur 1. Dies lässt sich im Rahmen der Erfindung wiederum beliebig mit den Häufigkeiten gemäß einer der Figuren 2 bis 5 kombinieren.

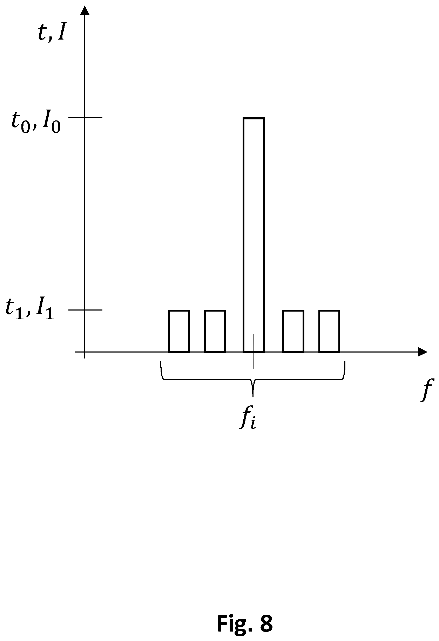

In der Figur 8 sind die Intensitätswerte bzw. Zeitdauern pro Frequenz als Balken mit teilweise unterschiedlicher Höhe und mit gleicher, endlicher Breite dargestellt, obwohl in der Praxis wiederum bevorzugt jeweils genau ein exakter Frequenzwert ausgewählt wird. Das markierte Intervall $f_i$ bezeichnet auch hier den Bereich, aus dem die Frequenzen $f_i$ ausgewählt werden, z.B. zwischen 5 MHz und 15 MHz. Die gezeigten Zeitdauern betragen bevorzugt $t_1$=1 ms und to=10 ms, und die Intensitäten liegen bevorzugt bei $I_0$=1 W/cm$^2$ und $I_1$=0,1 W/cm$^2$. Die Werte mit Index Null gehören zu der markierten mittleren Frequenz, die bevorzugt 10 MHz beträgt und der (Haupt-) Resonanzfrequenz des Transducers 10 (siehe Figur 1) entsprechen kann. Dadurch lässt sich der bereits angesprochene Effekt der stochastischen Resonanz ausnutzen.

Mit anderen Worten: Figur 8 zeigt (ohne Beschränkung) fünf distinkte Frequenzwerte bzw. entsprechend erzeugte Ultraschallwellen $U(f_i)$, von denen vier während einer gleichen Zeitdauer und/oder mit jeweils gleicher Intensität erzeugt werden. Die mittlere Frequenz wird dagegen mit eigener (höherer) Intensität und/oder eigener (längerer) Zeitdauer erzeugt. Dies lässt sich im Rahmen der Erfindung wiederum beliebig mit den Häufigkeiten gemäß einer der Figuren 2 bis 5 kombinieren.

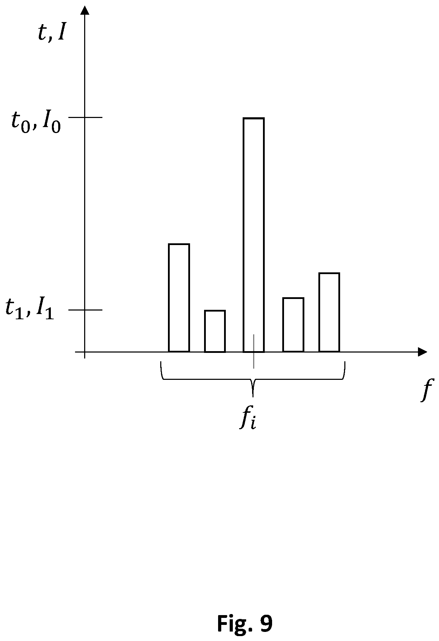

In der Figur 9 sind die Intensitätswerte bzw. Zeitdauern pro Frequenz als Balken mit teilweise unterschiedlicher Höhe und mit gleicher, endlicher Breite dargestellt, obwohl in der Praxis wiederum bevorzugt jeweils genau ein exakter Frequenzwert ausgewählt wird. Das markierte Intervall $f_i$ bezeichnet auch hier den Bereich, aus dem die Frequenzen $f_i$ ausgewählt werden, z.B. zwischen 5 MHz und 15 MHz. Die gezeigten Zeitdauern betragen bevorzugt zwischen $t_1$=1 ms und to=10 ms, und die Intensitäten liegen bevorzugt zwischen $I_0$=1 W/cm$^2$ und $I_1$=0,1 W/cm$^2$. Die markierte mittlere Frequenz beträgt bevorzugt 10 MHz und kann der (Haupt-) Resonanzfrequenz des Transducers 10 (siehe Figur 1) entsprechen. Auch so lässt sich der bereits angesprochene Effekt der stochastischen Resonanz ausnutzen.

Mit anderen Worten: Figur 9 zeigt (ohne Beschränkung) fünf distinkte Frequenzwerte bzw. entsprechend erzeugte Ultraschallwellen $U(f_i)$, von denen vier während einer Zeitdauer relativ kurzen Zeitdauer und/oder mit jeweils relativ geringer Intensität erzeugt werden. Die mittlere Frequenz wird dagegen mit eigener (höherer) Intensität und/oder eigener (längerer) Zeitdauer erzeugt. Die Auswahl von Intensität und/oder Zeitdauer kann zumindest teilweise zufällig erfolgen, bevorzugt nach Maßgabe durch die Zufallsfunktion 6 gemäß Figur 1.Dies lässt sich im Rahmen der Erfindung wiederum beliebig mit den Häufigkeiten gemäß einer der Figuren 2 bis 5 kombinieren.

## Patentansprüche

1. System (1) zur Erzeugung von Ultraschallwellen, insbesondere zur kosmetischen oder therapeutischen Behandlung, aufweisend:

   einen elektrisch angesteuerten Ultraschallkopf (5), der dazu ausgebildet ist, Ultraschallwellen $U(f_i)$ mit mehreren unterschiedlichen Frequenzen $f_i$ zu erzeugen, wobei die genannten unterschiedlichen Frequenzen $f_i$ eine Gruppe von Frequenzen bilden,
   einen Signalgenerator (4), der dazu ausgebildet ist, Signale $S(f_i)$ mit den genannten Frequenzen $f_i$ zu erzeugen und zur Umwandlung in Ultra-

schallwellen $U(f_i)$ an dem Ultraschallkopf (5) bereitzustellen,

und

eine Steuereinheit (3), die dazu ausgebildet ist, permanent oder zumindest während vorgegebener Zeitspannen zwischen den Frequenzen $f_i$ umzuschalten,

**dadurch gekennzeichnet, dass**

die Steuereinheit (3) dazu ausgebildet ist, nach Maßgabe einer Zufallsfunktion (6) zwischen allen oder zumindest einigen der genannten Frequenzen $f_i$ zufällig umzuschalten, wobei die einigen der genannten Frequenzen $f_i$ eine Untergruppe der genannten Gruppe bilden.

2. System (1) nach Anspruch 1, bei dem
die Gruppe eine Anzahl von mindestens drei, vorzugsweise mindestens fünf, höchst vorzugsweise mindestens zehn Frequenzen $f_i$ umfasst.

3. System (1) nach Anspruch 1 oder 2, bei dem
die Frequenzen $f_i$ in einem Bereich zwischen 3 MHz und 19 MHz, vorzugsweise zwischen 5 MHz und 15 MHz, liegen.

4. System (1) nach einem der Ansprüche 1 bis 3, bei dem
der Ultraschallkopf (5) einen Ultraschallwandler (10) aufweist, der etwa bei einer mittleren Frequenz aus der genannten Gruppe, vorzugsweise bei einer Frequenz von etwa 10 MHz, seine Resonanzfrequenz besitzt.

5. System (1) nach Anspruch 4, bei dem
die Steuereinheit (3) dazu ausgebildet ist, nach Maßgabe der Zufallsfunktion (6) zwischen allen oder zumindest einigen der genannten Frequenzen $f_i$ mit Ausnahme der genannten Resonanzfrequenz zufällig umzuschalten und die genannte Resonanzfrequenz nach einem regelmäßigen Muster zu wiederholen, insbesondere bei jedem zweiten oder dritten Frequenzwechsel.

6. System (1) nach einem der Ansprüche 1 bis 5, bei dem
die Steuereinheit (3) dazu ausgebildet ist, nach jeweils einer fest vorgegebenen oder einer ebenfalls zufällig gewählten Umschaltzeit t zwischen den Frequenzen $f_i$ umzuschalten, wobei vorzugsweise die Umschaltzeit t, insbesondere die zufällig gewählte Umschaltzeit t, zwischen 0,1 ms und 50 ms, vorzugsweise zwischen 0,1 ms und 10 ms, beträgt.

7. System (1) nach Anspruch 6, bei dem
die Umschaltzeit t mit der Frequenz f korreliert ist, wobei höhere Frequenzen längere Umschaltzeiten aufweisen.

8. System (1) nach einem der Ansprüche 1 bis 5, bei dem
die Steuereinheit (3) dazu ausgebildet ist, bei einer der Frequenzen eine erste Erzeugungsdauer (to) auszuwählen und bei den anderen Frequenzen eine von der ersten Erzeugungsdauer verschiedene, zweite Erzeugungsdauer $(t_1)$ auszuwählen, wobei vorzugsweise die zweite Erzeugungsdauer kürzer ist als die erste Erzeugungsdauer, wobei höchst vorzugsweise die eine der Frequenzen der Resonanzfrequenz gemäß Anspruch 4 entspricht und wobei insbesondere die erste Erzeugungsdauer (to) 10 ms und die zweite Erzeugungsdauer $(t_1)$ zwischen 0,1 und 1,0 ms beträgt.

9. System (1) nach einem der Ansprüche 1 bis 8, bei dem
die Steuereinheit (3) dazu ausgebildet ist, zusammen mit den Frequenzen $f_i$ auch eine Intensität I der Ultraschallwellen zu verändern, wobei vorzugsweise die Intensität I aus einem Intervall gewählt ist, das von 0 bis 3 $W/cm^2$, vorzugsweise von 0,5 bis 1,5 $W/cm^2$, reicht.

10. System (1) nach Anspruch 9, bei dem
die Steuereinheit (3) dazu ausgebildet ist, die Intensität I zumindest für einige der Frequenzen nach Maßgabe einer Zufallsfunktion (6) zu verändern.

11. System (1) nach Anspruch 9 oder 10, bei dem
die Steuereinheit (3) dazu ausgebildet ist, bei einer der Frequenzen eine erste Intensität $(I_0)$ auszuwählen und bei den anderen Frequenzen eine von der ersten Intensität verschiedene, zweite Intensität $(I_1)$ auszuwählen.

12. System (1) nach einem der Ansprüche 1 bis 11, bei dem
die Steuereinheit (3) dazu ausgebildet ist, alle Frequenzen mit im Wesentlichen gleicher Wahrscheinlichkeit oder Häufigkeit, p, auszuwählen, $p \propto f^0$.

13. System (1) nach einem der Ansprüche 1 bis 11, bei dem
die Steuereinheit (3) dazu ausgebildet ist, die Frequenzen mit einer Wahrscheinlich oder Häufigkeit, p, auszuwählen, die einer Gauß'schen Normalverteilung mit einem Maximum bei einer der Frequenzen entspricht.

14. System (1) nach einem der Ansprüche 1 bis 11, bei dem
die Steuereinheit (3) dazu ausgebildet ist, die Frequenzen mit einer Wahrscheinlich oder Häufigkeit, p, auszuwählen, die einer von dem Frequenzwert abhängigen mathematischen Funktion entspricht, wobei vorzugweise gilt $p \propto 1/f$ oder $p \propto f^2$ oder $p \propto f$ oder $p \propto f^2$.

**Fig. 1**

**Fig. 2**

**Fig. 3**

**Fig. 4**

$$f(x) = \frac{1}{x}$$

**Fig. 5**

**Fig. 6**

**Fig. 7**

Fig. 8

**Fig. 9**

**EUROPÄISCHER RECHERCHENBERICHT**

Nummer der Anmeldung

EP 24 21 8686

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2014/128863 A1 (DU SHU [US] ET AL) 8. Mai 2014 (2014-05-08) | 1,2,4,6, 8-11,13, 14 | INV. A61N7/00 |
| Y | * Absatz [0009] * | 3,7 | |
| A | * Absätze [0024] - [0035]; Abbildungen 1-2 * <br> * Absatz [0044]; Abbildung 3 * <br> * Absätze [0051] - [0069]; Abbildungen 7-8 * | 5 | |
| | ----- | | |
| X | US 5 720 710 A (TACHIBANA KATSURO [JP] ET AL) 24. Februar 1998 (1998-02-24) | 1,2,6,12 | |
| A | * Spalte 2, Zeile 60 - Spalte 3, Zeile 56; Abbildungen 1-2 * | 5 | |
| | ----- | | |
| Y | EP 3 031 439 B1 (WELLCOMET GMBH [DE]) 25. August 2021 (2021-08-25) <br> * Absatz [0011]; Abbildung 1 * <br> * Absätze [0024] - [0029] * | 3,7 | |
| | ----- | | RECHERCHIERTE SACHGEBIETE (IPC) |
| X | EP 2 799 112 A1 (ALPINION MEDICAL SYSTEMS CO [KR]) 5. November 2014 (2014-11-05) | 1,2,9-12 | |
| A | * Absätze [0009] - [0021] * <br> * Absatz [0032]; Abbildungen 3-6 * <br> * Absätze [0041] - [0048]; Abbildungen 7-8 * | 5 | A61N |
| | ----- | | |
| A | US 2023/330442 A1 (AGARWAL VIJAY [US] ET AL) 19. Oktober 2023 (2023-10-19) <br> * Absätze [0174] - [0196]; Abbildungen 1-8 * | 1-14 | |
| | ----- | | |

Der vorliegende Recherchenbericht wurde für alle Patentansprüche erstellt

| Recherchenort | Abschlußdatum der Recherche | Prüfer |
|---|---|---|
| Den Haag | 15. Mai 2025 | Schnurbusch, Daniel |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...........................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 24 21 8686

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-05-2025

| Im Recherchenbericht angeführtes Patentdokument | Datum der Veröffentlichung | Mitglied(er) der Patentfamilie | Datum der Veröffentlichung |
|---|---|---|---|
| US 2014128863 A1 | 08-05-2014 | CN 104780858 A | 15-07-2015 |
| | | EP 2916756 A1 | 16-09-2015 |
| | | US 2014128863 A1 | 08-05-2014 |
| | | US 2016235424 A1 | 18-08-2016 |
| | | US 2017296216 A1 | 19-10-2017 |
| | | WO 2014074273 A1 | 15-05-2014 |
| US 5720710 A | 24-02-1998 | DE 69425792 T2 | 15-02-2001 |
| | | EP 0634189 A2 | 18-01-1995 |
| | | JP 3415203 B2 | 09-06-2003 |
| | | JP H0724074 A | 27-01-1995 |
| | | US 5720710 A | 24-02-1998 |
| EP 3031439 B1 | 25-08-2021 | EP 2249762 A2 | 17-11-2010 |
| | | EP 3031439 A1 | 15-06-2016 |
| | | KR 20100126475 A | 01-12-2010 |
| | | KR 20150139976 A | 14-12-2015 |
| | | WO 2009109196 A1 | 11-09-2009 |
| | | WO 2009112181 A2 | 17-09-2009 |
| EP 2799112 A1 | 05-11-2014 | EP 2799112 A1 | 05-11-2014 |
| | | KR 20140105453 A | 01-09-2014 |
| | | WO 2013100235 A1 | 04-07-2013 |
| US 2023330442 A1 | 19-10-2023 | AU 2021321597 A1 | 09-03-2023 |
| | | CA 3185803 A1 | 10-02-2022 |
| | | CN 116209470 A | 02-06-2023 |
| | | EP 4192404 A2 | 14-06-2023 |
| | | IL 300335 A | 01-04-2023 |
| | | JP 2023537219 A | 31-08-2023 |
| | | KR 20230066556 A | 16-05-2023 |
| | | US 2023330442 A1 | 19-10-2023 |
| | | US 2023330443 A1 | 19-10-2023 |
| | | US 2023338754 A1 | 26-10-2023 |
| | | US 2024024649 A1 | 25-01-2024 |
| | | US 2025121214 A1 | 17-04-2025 |
| | | WO 2022032283 A2 | 10-02-2022 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3031439 B1